# EUROPEAN PATENT SPECIFICATION

(11) **EP 4 047 613 B1**
(45) Date of publication and mention of the grant of the patent: **09.12.2026**
(21) Application number: 22158350.3
(22) Date of filing: 15.06.2016
(51) Int. Cl.: G16H 20/30, G16H 40/63, G16H 50/20

(54) **CONTEXT-AWARE SYSTEM FOR PROVIDING FITNESS INFORMATION**
KONTEXTBEWUSSTES SYSTEM ZUR BEREITSTELLUNG VON FITNESSINFORMATIONEN
SYSTÈME SENSIBLE AU CONTEXTE POUR FOURNIR DES INFORMATIONS DE FITNESS

(30) Priority: 17.07.2015 US 201562193685 P; 29.07.2015 US 201514812379
(43) Date of publication of application: 24.08.2022
(62) Document number(s) of the earlier application(s) in accordance with Art. 76 EPC: 16735761.5 / 3 326 090
(73) Proprietor: Google LLC, Mountain View, CA 94043 (US)
(72) Inventor: SOULOS, Paul, Mountain View, 94043 (US); GALE, Allyson, Mountain View, 94043 (US)
(74) Representative: Trichard, Louis

(56) References cited:
- WO-A1-2014/093856
- US-A1- 2013 231 574
- US-A1- 2014 143 064

## Description

### BACKGROUND

Some people may sit or remain mostly sedentary for what some health experts consider to be an unhealthy amount of time. To help users to maintain more healthy and active lifestyles, various types of mobile and wearable computing devices exist for tracking user activity and notifying the user when he or she has been sitting for too long or for notifying the user to become active at pre-determined times in the day.

Even though a person may have been sitting for a long time, in some situations, it may be impractical to stand up and move around. For example, a user may have to stay seated during a meeting or when riding in a car or train. If an activity tracking device generates a notification to alert the user to become active while the user is in a situation in which he or she is unable to move around, the user may simply dismiss the notification and forget to become active at a later time.

WO 2014/093856 A1 discloses that athletic performance monitoring and tracking may provide multiple ways in which to track athletic movement and activity. Workouts may also be tagged with various parameters including mood, weather, terrain, athletic equipment, friends used and the like. Recommended activities to be performed or a recommended time to perform an activity may be determined based on a user's schedule, weather or conditions forecasts, or a location of the user or the potential activity.

### SUMMARY

The matter for protection is defined by the appended claims. Where subject matter does not fall within the scope of the appended claims, it is to be understood as an example useful for understanding the invention.

### BRIEF DESCRIPTION OF DRAWINGS

FIG. 1 is a conceptual diagram illustrating an example system configured to recommend physical activities for a user of a computing device to perform at appropriate times, in accordance with one or more aspects of the present disclosure.
FIG. 2 is a block diagram illustrating an example computing device configured to recommend physical activities for a user of the computing device to perform at appropriate times, in accordance with one or more aspects of the present disclosure.
FIG. 3 is a block diagram illustrating an example computing device that outputs graphical content for display at a remote device, in accordance with one or more techniques of the present disclosure.
FIGS. 4 and 5 are flowcharts illustrating example operations of an example computing device configured to recommend physical activities for a user of the computing device to perform at appropriate times, in accordance with one or more aspects of the present disclosure.

### DETAILED DESCRIPTION

In general, techniques of this disclosure may enable a computing device to determine, based on contextual information, that a user has been physically inactive for a prolonged period of time and, in response, determine a recommended physical activity the user could perform at the current time. For example, the computing device may analyze movement data associated with the computing device for identifying sedentary periods associated with a user. Responsive to determining that the user of the computing device has not been physically active for some time, the computing device determines whether "now" (e.g., the current time) is an appropriate time to recommend that the user become physically active.

The device obtains contextual information associated with the computing device and infers, given the current context, a recommended physical activity that the user could perform at the current time to become more physically active. The computing device may output the recommended physical activity as a notification (e.g., a graphical alert, a tactile alert, or an audible alert) for nudging the user into performing the recommended physical activity at the current time. In some instances, the device may determine that for the given context, the current time may not be a good time for the user to become physically active (e.g., if the device infers that the user is driving a car, sitting in a meeting or lecture, or performing some other activity that may not be conducive for brief physical activity or exercise) and may refrain from recommending the physical activity, or in some instances, defer the recommendation until a later time when the user is more likely able to become physically active.

Over time, by causing the computing device to output more and more intelligent notifications of suggested physical activities, the computing device may coach the user into becoming more physically active. Moreover, the computing device may perform these operations automatically without, for example, requiring such operations being initiated by the user, thereby reducing the amount of user input, effort, and time required for finding ways to be more physically active.

Throughout the disclosure, examples are described wherein a computing device and/or computing system may analyze information (e.g., locations, speeds, accelerations) associated with the computing device and information (e.g., communications, calendars, files and notes) associated with the user of the computing device only if the computing device and/or the computing system receives explicit permission from the user of the computing device to analyze the information. For example, in situations discussed below in which the computing device and/or computing system may collect or may make use of information associated with the user and the computing device, the user may be provided with an opportunity to provide input to control whether programs or features of the computing device and/or computing system can collect and make use of user information (e.g., information about a user's e-mail, a user's social network, social actions or activities, profession, a user's preferences, or a user's past and current location), or to dictate whether and/or how the computing device and/or computing system may receive content that may be relevant to the user. In addition, certain data may be treated in one or more ways before it is stored or used by the computing device and/or computing system, so that personally-identifiable information is removed. For example, a user's identity may be treated so that no personally identifiable information can be determined about the user, or a user's geographic location may be generalized where location information is obtained (such as to a city, ZIP code, or state level), so that a particular location of a user cannot be determined. Thus, the user may have control over how information is collected about the user and used by the computing device and/or computing system.

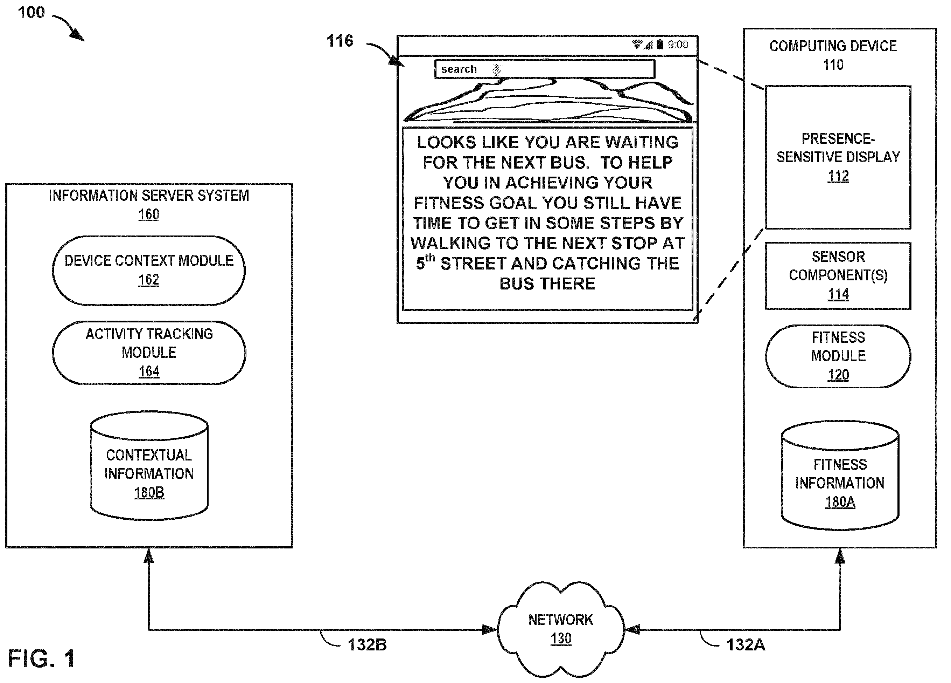

FIG. 1 is a conceptual diagram illustrating system 100 as an example system configured to recommend physical activities for a user of a computing device to perform at appropriate times, in accordance with one or more aspects of the present disclosure. System 100 includes computing device 110, information server system 160, and network 130. Although shown as separate devices, in some examples, computing device 110 and information server system 160 represent a single computing device. In some examples, computing device 110 may include at least some of the functions or capability of information server system 160, and vice versa.

Network 130 represents any public or private communication network, for instance, a cellular, Wi-Fi, and/or other type of network for transmitting data between computing devices. Computing device 110 and information server system 160 may send and receive data across network 130 using any suitable communication techniques. For example, computing device 110 may be operatively coupled to network 130 using network link 132A and information server system 160 may be operatively coupled to network 130 by network link 132B. Network 130 may include network hubs, network switches, network routers, and other network devices that are operatively inter-coupled thereby providing for the exchange of information between computing device 110 and information server system 160. In some examples, network links 132A and 132B may be Ethernet, Asynchronous Transfer Mode (ATM) network, or other network connections and such connections may be wireless and/or wired connections.

Information server system 160 represents any suitable remote computing system, such as one or more desktop computers, laptop computers, mainframes, servers, or cloud computing systems capable of sending and receiving information across network link 132B to network 130. In some examples, information server system 160 represents a cloud computing system that provides one or more services through network 130. One or more computing devices, such as computing device 110, may access the one or more services provided by the cloud via network 130. For example, computing device 110 may store and/or access data in the cloud by communicating, via network 130, with information server system 160.

Information server system 160 includes device context module 162, activity tracking module 164, and contextual information data store 180B. Modules 162 and 164 may perform operations described using software, hardware, firmware, or a mixture of hardware, software, and firmware residing in and/or executing at information server system 160. Information server system 160 may execute information modules 162 and 164 with multiple processors or multiple devices. Information server system 60 may execute modules 162 and 164 as a virtual machine executing on underlying hardware. Modules 162 and 164 may execute as a service of an operating system or computing platform. Modules 162 and 164 may execute as one or more executable programs at an application layer of a computing platform.

Data store 180B represents any suitable storage medium for storing data, specifically, data related to contextual information. Information server system 160 may collect contextual information associated with computing devices, such as computing device 110, and store the collected contextual information at data store 180B. Information server system 160 may provide access to the contextual information stored at data store 180B as a cloud based, data-access service to devices connected to network 130, such as computing device 110. When data store 180B contains contextual information associated with individual users or when the information is genericized across multiple users, all personally-identifiable-information such as name, address, telephone number, and/or e-mail address linking the information back to individual people may be removed before being stored at information server system 160. Information server system 160 may further encrypt the information stored at data store 180B to prevent access to any information stored therein. In addition, information server system 160 may only store contextual information associated with users of computing devices if those users affirmatively consent to such collection of information. Information server system 160 may further provide opportunities for users to withdraw consent and in which case, information server system 160 may cease collecting or otherwise retaining the contextual information associated with that particular user.

As used throughout the disclosure, the term "contextual information" is used to describe information that can be used by a computing system and/or computing device, such as information server system 160 and computing devices 110, to define one or more environmental characteristics associated with computing devices and/or users of computing devices. In other words, contextual information represents any data that can be used by a computing device and/or computing system to determine a "user context" indicative of the circumstances that form the experience the user undergoes (e.g., virtual and/or physical) for a particular location at a particular time.

Examples of contextual information include past, current, and future physical locations, degrees of movement, magnitudes of change associated with movement, weather conditions, traffic conditions, patterns of travel, patterns of movement, application usage, calendar information, purchase histories, Internet browsing histories, and the like. In some examples, contextual information may include sensor information obtained by one or more sensors (e.g., gyroscopes, accelerometers, proximity sensors) of computing devices, such as computing device 110, radio transmission information obtained from one or more communication units and/or radios (e.g., global positioning system (GPS), cellular, Wi-Fi) of computing devices, information obtained by one or more input devices (e.g., cameras, microphones, keyboards, touchpads, mice) of computing devices, and network/device identifier information (e.g., a network name, a device internet protocol address). In some examples, contextual information may include communication information such as information derived from e-mail messages, text messages, voice mail messages or voice conversations, calendar entries, task lists, social media network related information, and any other information about a user or computing device that can support a determination of a user context.

Context module 162 may perform operations for determining a context associated with users of computing devices, such as a user of computing device 110. Context module 162 may process and analyze contextual information stored at data store 180B, and based on the contextual information, define a user context specifying the state or physical operating environment a user of computing devices 110. In other words, context module 162 may process contextual information received from computing devices 110 that information server system 160 stores at data store 180B to generate a user context of the user of computing devices 110 that specifies one or more characteristics associated with the user of computing devices 110 and his or her physical surroundings at a particular time (e.g., location, name of establishment, street address, and/or type of place, building, weather conditions, traffic conditions, calendar information, meeting information, event information). In some examples, context module 162 may determine a physical location associated with computing device 110 based on the contextual information stored at data store 180B and update the physical location as context module 162 detects changes in the contextual information (e.g., based on sensor information indicative of movement over time).

Context module 162 may maintain contextual histories associated with the user of computing device 110 and store the contextual histories as contextual information at data store 180B. For example, context module 162 may periodically update a location of computing device 110 and store the location along with a day and time at data store 180B as a location history. Context module 162 may share the location information with activity tracking module 164 and fitness module 120 of computing device 110 to predict, infer, or confirm whether a user of computing device 110 is presently engaged in physical or sedentary activity and, further, to predict whether the user could perform (e.g., with ease and without suffering from embarrassment or social distain) one or more physical activities at a particular time.

Activity tracking module 164 may determine, based on contextual information, one or more activities being performed by a user at a particular time. For example, activity tracking module 164 may execute a rules-based algorithm or a machine learning system that predicts what a user of a computing device is doing at a given time. The rules based algorithm or a machine learning system may be based on various observations about user behavior for different contexts.

Activity tracking module 164 may query context module 162 for an indication of a context associated with a user of computing device 110 and responsive to inputting the context into a rules-based algorithm or a machine learning system, receive an output indicative of one or more activities that the user may be performing given the context. For example, if a context received from device context module 162 defines a speed and a location of computing device 110 that coincides with the speed of typical train when the train moves on a particular rail track, then the system may predict, based on the rules, that the user is likely riding in (or on) a particular train. In other examples, if the context received from context module 162 indicates that the location of computing device 110 corresponds to a work location of a user and that the location has not changed for some period of time, the system may predict, based on the rules, that the user is likely sitting or standing at his or her work desk. In still other examples, if the context received from context module 162 indicates that the location of computing device 110 corresponds to bus stop of a bus line that the user normally takes to go home at the current time, the system may predict, based on the rules, that the user is likely waiting for a bus to return home.

In some examples, activity tracking module 164 may determine a respective score, probability, or other degree of likelihood associated with each of the one or more activities that indicates how likely or unlikely that the user is actually performing the activity for the particular time. For instance, activity tracking module 164 may determine with a ninety-five percent confidence that a user is driving a car, riding in a train, sitting at a desk, or performing some other activity.

Activity tracking module 164 may provide an application programming interface (API) from which computing devices, such as computing device 110, can query activity tracking module 164 for a current activity being performed by a user at a particular time. For example, responsive to receiving a query, through network 130 via network link 132B, from computing device 110 of the current activity being performed by the user of computing device 110, activity tracking module 164 may output, via network link 132B and through network 130, an indication (e.g., data, a message, a signal) that indicates which activity the user is likely performing at the current time and/or a probability, score, or other degree of likelihood or confidence level that the system has that the user is performing the activity. For example, activity tracking module 164 may respond to a query from computing device 110 with a message indicating that the system predicts, with a high degree of confidence, the user is waiting for a bus at a bus stop.

In the example of FIG. 1, computing device 110 is a mobile computing device. However, in other examples, computing device 110 may be a tablet computer, a personal digital assistant (PDA), a laptop computer, a portable gaming device, a portable media player, an e-book reader, a watch, a television platform, an automobile navigation system, a wearable computing device (e.g., a headset device, watch device, eyewear device, a glove device), or other type of computing device.

As shown in FIG. 1, computing device 110 includes presence-sensitive display (PSD) 112. PSD 112 of computing device 110 may function as an input device for computing device 110 and as an output device. PSD 112 may be implemented using various technologies. For instance, PSD 112 may function as an input device using a presence-sensitive input component, such as a resistive touchscreen, a surface acoustic wave touchscreen, a capacitive touchscreen, a projective capacitance touchscreen, a pressure sensitive screen, an acoustic pulse recognition touchscreen, or another presence-sensitive display technology. PSD 112 may function as an output (e.g., display) device using any one or more display components, such as a liquid crystal display (LCD), dot matrix display, light emitting diode (LED) display, organic light-emitting diode (OLED) display, e-ink, or similar monochrome or color display capable of outputting visible information to a user of computing device 110.

Computing device 110 also includes one or more sensor components 114. Numerous examples of sensor components 114 exist and include any input component configured to obtain environmental information about the circumstances surrounding computing device 110 and/or physiological information that defines the activity state and/or physical well-being of a user of computing device 110. For example, sensor components 114 may include movement sensors (e.g., accelerometers), temperature sensors, position sensors (e.g., a gyro), pressure sensors (e.g., a barometer), proximity sensors (e.g., an inferred sensor), ambient light detectors, heart-rate monitors, and any other type of sensing component. Computing device 110 may use sensor components 114 to obtain contextual information associated with computing device 110 and a user. In some examples, fitness module 120 of computing device 110 may rely on the sensor information obtained by sensor components 114. In some examples, computing device 110 may relay information obtained from sensor components 114 to information server system 160 (e.g., for storage and subsequent retrieval from data store 180B).

Computing device 110 includes fitness information data store 180A which represents any suitable storage medium for storing data, specifically, data related to fitness information. In general, the term "fitness information" refers to any information that computing device 10 may use to determine a recommended physical activity that a person may perform, for a particular context (e.g., to achieve a fitness goal). Examples of fitness goals include a maximum or minimum heart rate level, a maximum or minimum amount of time spent sitting down or otherwise remaining sedentary, a body weight, a quantity of footsteps taken by a person over a time duration, a distance traveled over a time duration, and/or an amount of time spent by a person performing a physical activity or exercise.

The fitness information stored at data store 180A may be generic information (e.g., normalized across multiple people) and/or may be specific information associated with particular person, such as a user of computing device 110. For example, computing device 110 may store information related to one or more fitness goals associated with averaged users of multiple computing devices, including computing device 110; and computing device 110 may store information related to one or more fitness goals associated with a particular user of computing device 110. As described below, fitness module 120 may access the fitness information data stored at data store 180A.

Although data store 180A may contain fitness information associated with individual users, the information may be treated such that all personally-identifiable-information (such as name, address, telephone number, e-mail address) linking the information back to individual people may be removed before being stored at computing device 110. In addition, computing device 110 may only store fitness information associated with users of computing device 110 if those users affirmatively consent to such collection of information. Computing device 110 may further provide opportunities for users to remove such consent and in which case, computing device 110 may cease collecting fitness and contextual information associated with that particular user.

Fitness information data store 180A may store information related to one or more types of physical activities or exercises (e.g., bicycling, walking, running, jogging, canoeing, kayaking, roller skating) that a person may perform in order to be more physically active at a particular time. For example, fitness information data store 180A may store fitness information about bicycle riding, such as an average amount of energy expended by a person per unit of distance traveled while the person rides a bicycle. Other examples of fitness information may include weather information (e.g., temperature, humidity) indicative of a stated and/or predicted preferred weather condition that a person prefers while walking. In some examples, fitness information data store 180A may store types of physical activities or exercises according to pre-defined contexts. For example, fitness information data store 180A may include a matrix of different contexts and corresponding activities. A row of the matrix may be associated with a particular context and each column may be associated with a particular activity. Accordingly, the matrix may define, for each of the different context, which types of activities that could be performed, in those different contexts.

Other examples of the types of information stored at data store 180A include information about a person's stated or inferred fitness goals, workout history, current exercise performance, historical fitness performance or historical activity information (e.g., average walking speed, jogging speed, heart rates, etc.). Still other types of information stored at data store 180A may include information that indicates a person's daily activity level for a current day, current month, current year, or a history of the person's daily activity levels over multiple days, months, or years. For example, data store 180A may include an entry that indicates a quantity of steps taken by the user for the current day or a projected quantity of calories burned by the user on the particular day.

The fitness information may be organized and searchable within data store 180A (e.g., according to physical activity or exercise type, individual person's names, etc.). Computing device 110 may access the data within data store 180A, for instance, by executing a query command related to one or more potential physical activities that could be performed for a particular context. Responsive to the query command, information server system 160 may obtain information from data store 180A related to the one or more recommended physical activities from the one or more potential physical activities that best fit a user's lifestyle or preferred exercising habits that computing device 110 infers from the fitness information in data store 180A, or the one or more recommended physical activities that may otherwise assists the user in achieving his or her fitness goals (e.g., for becoming more active). Computing device 110 may use the information retrieved from data store 180A to determine whether or not to recommend a particular physical activity as a recommended physical activity for a user of computing device 110 to perform at the current time.

Computing device 110 may include fitness module 120 for determining, based on contextual information, whether a user of computing device 110 has been physically inactive for a prolonged period of time and, if so, determine a recommended physical activity the user could perform at the current time to become more physically active. For example, as described below, fitness module 120 may rely on context module 162 to figure out a current context associated with the user (e.g., where a user is located and characteristics of the environment around the user at that location). Then, fitness module 120 may figure out one or more exercises that can possibly be performed in the current context (e.g., what type of exercises the user may perform at the location given the characteristics of the environment). But before recommending any of the exercises to the user, fitness module 120 may then rely on activity tracking module 164 to infer what the user is really doing in the particular context (e.g., at that location and in that particular environment) so as to subsequently determine whether fitness module 120 should recommend any of the exercises. In other words, fitness module 120 may determine whether it is practical for the user to perform, without interfering with the activity being performed by the user in the current context, one of the recommended exercises that is suited for the context.

Fitness module 120 may perform operations described using software, hardware, firmware, or a mixture of hardware, software, and firmware residing in and/or executing at computing device 110. Computing device 110 may execute fitness module 120 with one or more processors. Computing device 110 may execute fitness module 120 as a virtual machine executing on underlying hardware. Fitness module 120 may execute as a service or component of an operating system or computing platform. Fitness module 120 may execute as one or more executable programs at an application layer of a computing platform.

Fitness module 120 may cause PSD 112 to present a graphical user interface from which a user can monitor, track, and be apprised of information related to his or her physical activity and exercise performance. For instance, screen shot 116 shows an example of the type of graphical user interface that fitness module 120 may cause PSD 112 to display for alerting or notifying a user about fitness related information. In the example of FIG. 1, screen shot 116 illustrates a notification or an "information card" as one example graphical element that fitness module 120 may present at PSD 112. These so called information cards may present fitness information that is relevant for a current context of computing device 110. Fitness module 120 may cause PSD 112 to present an information card, for instance, in response to determining that the user would like to be nudged into performing an exercise at the current time.

Fitness module 120 may detect movement associated with computing device 110 based on sensor information obtained from sensor components 114 to determine periods of idleness or general inactivity. For example, fitness module 120 may obtain accelerometer information from an accelerometer of sensor components 114 and determine whether movement inferred from the accelerometer indicates that the person is moving (e.g., walking, jogging, etc.) or whether the movement is only slight and therefore indicates that the user is sedentary (e.g., sitting, sleeping, standing still, etc.).

In some examples, fitness module 120 may compare the sensor information obtained from sensor components 114 to one or more activity thresholds that fitness module 120 uses to determine whether a user of computing device 110 is currently active and not in need of a nudge to move around or whether the user is inactive (e.g., sleeping, sitting, etc.) and might be in need of a recommended exercise period. For example, an activity threshold may correspond to a pattern of movement often detected by computing device 110 when a user is walking, jogging, bicycling, etc. Fitness module 120 may determine that if the detected movement based on the sensor information obtained from sensor components 114 sufficiently corresponds to the pattern of movement defined by the activity threshold, that the user of computing device 110 is active. Fitness module 120 may determine that if the detected movement does not satisfy the activity threshold indicative of the user of the computing device 110 being in a physically active state, that the user of computing device 110 is inactive.

As used herein, the term "physically active state" refers to moments when a user is walking, standing, bicycling, swimming, exercising, or otherwise moving in such a way as to be considered "physically active." Physically active may be defined by heart rate, perspiration, breathing rate, or some other physiological condition that indicates the user is not resting. Conversely, the term "physically inactive state" refers to moments when the user is sitting, lying, sleeping, floating, relaxing, or otherwise moving in such a way as to be considered "physically inactive." Physically inactive may be defined by heart rate, perspiration, breathing rate, or some other physiological condition that indicates the user is resting.

Fitness module 120 may rely on activity thresholds that are based on actual, modeled, predicted, or otherwise derived patterns of movement. In some examples, a machine learning system (or other type of predictive or artificial intelligence type model) of fitness module 120, may generate and access the patterns of movement to later infer, predict, or otherwise determine periods of time when the user of computing device 110 is likely in an active state or not.

Fitness module 120 of computing device 110 may collect, analyze, and otherwise maintain the fitness information stored at data store 180A. The information maintained by fitness module 120 at data store 180A may be provided explicitly (e.g., from a user through user interaction with fitness module 120 and a graphical user interface displayed at PSD 112). Alternatively, or additionally, the information maintained by fitness module 120 at data store 180A may be implicitly provided (e.g., based on movement of computing device 110 detected as the user performs one or more physical activities or exercises while carrying computing device 110).

Responsive to determining that movement associated with computing device 110 does not satisfy an activity threshold indicative of a user of the computing device being in a physically active state, fitness module 120 may determine, based at least in part on contextual information associated with computing device 110, a recommended physical activity for the user of computing device 110 to perform (e.g., at the current time). Said differently, in order to encourage a user of computing device 110 to become more active, fitness module 120 identifies one or more physical activities that the user could perform instead of remaining mostly sedentary or idle.

For example, fitness module 120 may issue a query to context module 162 of information server system 160 for a current context of computing device after detecting a prolonged period of inactivity associated with the user (e.g., thirty minutes, one hour, one day, etc.). In response to the query, fitness module 120 may receive an indication (e.g., data, message, signal, and the like) that defines for fitness module 120, the current context of computing device 110. For example, fitness module 120 may receive information from context module 162 indicating that the user is likely standing along a street or at an intersection that is located at or near a bus stop. Based on the information received from context module 162, fitness module 120 may determine one or more exercises that the user could perform while at the bus stop.

Fitness module 120 may query the context obtained from context module 162 (e.g., "bus stop") at data store 180A and, in response to the query (e.g., based on a lookup of information at the matrix maintained at data store 180A), receive an indication of one or more exercises that a user of computing device 110 could perform given the current context (e.g., at the bus stop). In some examples, a machine learning system (or other type of predictive or artificial intelligence type model) of fitness module 120, may infer, predict, or otherwise determine recommended exercises based on a context of computing device 110. For example, the machine learning system may maintain a rule that defines "squatting in place" as a recommended exercise that a user could perform while standing at or near a bus stop. In some examples, the machine learning system may maintain a rule that defines "walking, instead of riding, to the next bus stop" as a recommended exercise that a user could perform when fitness module 120 learns that the user happens to be standing at or near a bus stop. In other words, the machine learning system may determine one or more exercises that the user could perform while at the bus stop.

Further responsive to determining that movement associated with computing device 110 does not satisfy an activity threshold indicative of a user of the computing device being in a physically active state, fitness module 120 may determine, based on contextual information associated with computing device 110, a current activity associated with the user of computing device 110. Based on the current activity, fitness module 120 may determine whether it should recommend any of the one or more exercises that the user could perform while at the bus stop.

For example, fitness module 120 may issue a query to activity tracking module 164 for a current activity likely being performed by a user of computing device 110 at a current time. Activity tracking module 164 may query device context module 162 for a defined current context of computing device 110 and determine, based on the current context, one or more activities that a user of computing device 110 may be performing at the current time. For example, the rules based algorithm or machine learning system may receive the current context of computing device 110 as input and in response, output an indication of the one or more activities that the system infers about user behavior for the current context. In some examples, activity tracking module 164 may rely on calendar information, communication information, and/or other contextual information associated with a user to infer which activity the user may be performing at the current time.

In the example of FIG. 1, activity tracking module 164 may infer from the context associate with computing device 110, and from potentially other information associated with the user, that the user is "commuting home" after determining that the user is waiting at or near bus stop at a typical time of day that he or she would normally wait to take the bus home from work. Activity tracking module 164 may output an indication to fitness module 120 that the user is likely performing the activity of commuting home from a bus stop near his or her work. In some examples, activity tracking module 164 may determine a respective score, probability, or other degree of likelihood that indicates how likely or unlikely that the user is commuting home.

Further responsive to determining that movement associated with computing device 110 does not satisfy an activity threshold indicative of a user of computing device 110 being in a physically active state, and also responsive to determining that a degree of likelihood that the recommended physical activity can be performed concurrently with the current activity satisfies a probability threshold, fitness module 120 may output a notification of the recommended physical activity. For example, in response to determining that "walking to the next bus stop" is a recommended exercise when the user is located at or near a bus stop, fitness module 120 may determine whether the user has sufficient time to walk to the next stop and still catch the bus home to perform the inferred activity of "commuting home." In other words, fitness module 120 may determine whether any of the recommended exercises that the user could perform while at the bus stop can be performed at the bus stop without interfering with the user's commute home.

Fitness module 120 may determine (e.g., based on an Internet query for information) the location of the next stop of the bus and the estimated time of arrival for the bus at the next stop. Fitness module 120 may input the location of the next stop into a map or navigation application executing at computing device 110 and in response, receive indication of an estimated duration of time for the user of computing device 110 to walk to the next stop. In some examples, fitness module 120 may query other applications for information for discerning whether a recommended physical activity could be performed concurrently with a current activity.

Fitness module 120 may determine, based on fitness information stored at data store 180A, whether, given the average walking speed of the user, the use will likely arrive at the next stop in time to catch the bus there. For instance, fitness module 120 may determine that a greater than fifty percent chance exists that the user can walk to the next stop and still catch the bus home in response to determining that the estimated arrival time of the user at the next stop is less than the expected arrival time of the bus at the next stop by one minute or more.

Responsive to determining that the user will likely arrive at the next stop with five minutes to spare before the bus stops there, fitness module 120 may output a notification as a graphical indication that the user could gain some physical activity points by walking to the next stop instead of waiting at the current stop. In other words, fitness module 120 may determine that the user can with a degree of certainty walk to the next bus stop without interfering with the user's commute home. For example, fitness module 120 may cause presence-sensitive display to output a graphical notification, such as the notification displayed in screenshot 116.

Over time, by causing a computing device to output more and more intelligent notifications of suggested physical activities, the described techniques may enable a computing device to coach a user into becoming more physically active. Moreover, the described techniques may enable a computing device to perform these operations automatically without, for example, requiring such operations be initiated by the user thereby reducing the amount of user input, effort, and time required for finding ways to be more physically active. For example, if the computing device detects walking to the suggested bus stop and riding a bus from the suggested bus stop to the user's home, the computing device may increase the weighting or priority of the rule "walking, instead of riding, to the next bus stop" to maintain or increase the likelihood that the corresponding information card will be presented. Conversely, if the computing device fails to detect walking to the suggested bus stop and instead detects riding a bus from the original bus stop to the user's home, the computing device may decrease the weighting or priority of the rule "walking, instead of riding, to the next bus stop" to increase the likelihood that a different information card, such as "consider doing 10 squats" will be presented. As such, a user can interact with a computing device to track fitness progress and be alerted to become more active, without actually having to provide input at the computing device directly. In other words, the computing device may receive implicit input about the user. By not requiring the user to provide input to track fitness progress and set up alerts for becoming active, the computing device may perform fewer operations related to receiving the user input and therefore, consume less electrical power.

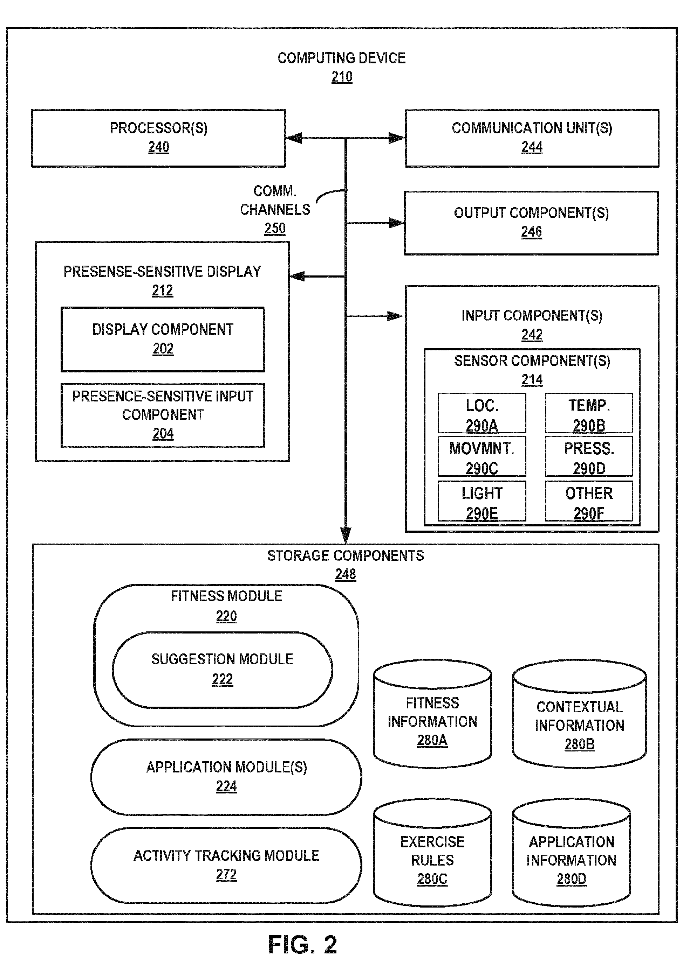

FIG. 2 is a block diagram illustrating computing device 210 as an example computing device configured to recommend physical activities for a user of the computing device to perform, at appropriate times, in accordance with one or more aspects of the present disclosure. Computing device 210 of FIG. 2 is described below within the context of computing device 110 and system 100 FIG. 1. Computing device 210 of FIG. 2 in some examples represents an example of computing device 110 of FIG. 1. In other examples, computing device 210 represents an example of system 100 of FIG. 1. FIG. 2 illustrates only one particular example of computing device 210, and many other examples of computing device 210 may be used in other instances and may include a subset of the components included in example computing device 210 or may include additional components not shown in FIG. 2.

As shown in the example of FIG. 2, computing device 210 includes presence-sensitive display 212, one or more processors 240, one or more input components 242, one or more communication units 244, one or more output components 246, and one or more storage components 248. Presence-sensitive display (PSD) 212 includes display component 202 and presence-sensitive input component 204. Input components 242 include sensor components 214.

Storage components 248 of computing device 200 also includes fitness module 220, one or more application modules 224, and activity tracking API module 272. Fitness module 220 also includes suggestion module 222. Additionally, storage components 248 include fitness information data store 280A, contextual information data store 280B, exercise rules data store 280C, and application information data store 280D (which exists either as a separate data store or as a subset of contextual information data store 280B) . Collectively, data stores 280A-280D may be referred to as "data stores 280".

Communication channels 250 may interconnect each of the components 202, 204, 212, 214, 220, 222, 224, 272, 240, 242, 244, 246, 248, and 280 for inter-component communications (physically, communicatively, and/or operatively). In some examples, communication channels 250 may include a system bus, a network connection, an inter-process communication data structure, or any other method for communicating data.

One or more input components 242 of computing device 210 may receive input. Examples of input are tactile, audio, and video input. Input components 242 of computing device 200, in one example, includes a presence-sensitive display, touch-sensitive screen, mouse, keyboard, voice responsive system, video camera, microphone or any other type of device for detecting input from a human or machine. One or more input components 242 include one or more sensor components 214. Numerous examples of sensor components 214 exist and include any input component configured to obtain environmental information about the circumstances surrounding computing device 210 and/or physiological information that defines the activity state and/or physical well-being of a user of computing device 210. For instance, sensor components 214 may include one or more location sensors 290A (GPS components, Wi-Fi components, cellular components), one or more temperature sensors 290B, one or more movement sensors 290C (e.g., accelerometers, gyros), one or more pressure sensors 290D (e.g., barometer), one or more ambient light sensors 290E, and one or more other sensors 290F (e.g., microphone, camera, infrared proximity sensor, hygrometer, and the like).

One or more output components 246 of computing device 210 may generate output. Examples of output are tactile, audio, and video output. Output components 246 of computing device 210, in one example, includes a presence-sensitive display, sound card, video graphics adapter card, speaker, cathode ray tube (CRT) monitor, liquid crystal display (LCD), or any other type of device for generating output to a human or machine.

One or more communication units 244 of computing device 210 may communicate with external devices via one or more wired and/or wireless networks by transmitting and/or receiving network signals on the one or more networks. Examples of communication unit 244 include a network interface card (e.g. such as an Ethernet card), an optical transceiver, a radio frequency transceiver, a GPS receiver, or any other type of device that can send and/or receive information. Other examples of communication units 244 may include short wave radios, cellular data radios, wireless network radios, as well as universal serial bus (USB) controllers.

Presence-sensitive display (PSD) 212 of computing device 200 includes display component 202 and presence-sensitive input component 204. Display component 202 may be a screen at which information is displayed by PSD 212 and presence-sensitive input component 204 may detect an object at and/or near display component 202. As one example range, presence-sensitive input component 204 may detect an object, such as a finger or stylus that is within two inches or less of display component 202. Presence-sensitive input component 204 may determine a location (e.g., an (x,y) coordinate) of display component 202 at which the object was detected. In another example range, presence-sensitive input component 204 may detect an object six inches or less from display component 202 and other ranges are also possible. Presence-sensitive input component 204 may determine the location of display component 202 selected by a user's finger using capacitive, inductive, and/or optical recognition techniques. In some examples, presence-sensitive input component 204 also provides output to a user using tactile, audio, or video stimuli as described with respect to display component 202. In the example of FIG. 2, PSD 212 presents a user interface (such as user interface screen shot 116 of FIG. 1).

While illustrated as an internal component of computing device 210, presence-sensitive display 212 may also represent and external component that shares a data path with computing device 210 for transmitting and/or receiving input and output. For instance, in one example, PSD 212 represents a built-in component of computing device 210 located within and physically connected to the external packaging of computing device 210 (e.g., a screen on a mobile phone). In another example, PSD 212 represents an external component of computing device 210 located outside and physically separated from the packaging of computing device 210 (e.g., a monitor, a projector, etc. that shares a wired and/or wireless data path with a tablet computer).

PSD 212 of computing device 210 may receive tactile input from a user of computing device 110. PSD 210 may receive indications of the tactile input by detecting one or more gestures from a user of computing device 210 (e.g., the user touching or pointing to one or more locations of PSD 212 with a finger or a stylus pen). PSD 212 may present output to a user. PSD 212 may present the output as a graphical user interface (e.g., as graphical screen shot 116), which may be associated with functionality provided by computing device 210. For example, PSD 212 may present various user interfaces of components of a computing platform, operating system, applications, or services executing at or accessible by computing device 210 (e.g., an electronic message application, a navigation application, an Internet browser application, a mobile operating system, etc.). A user may interact with a respective user interface to cause computing devices 210 to perform operations relating to a function.

PSD 212 of computing device 210 may detect two-dimensional and/or three-dimensional gestures as input from a user of computing device 210. For instance, a sensor of PSD 212 may detect a user's movement (e.g., moving a hand, an arm, a pen, a stylus, etc.) within a threshold distance of the sensor of PSD 212. PSD 212 may determine a two or three dimensional vector representation of the movement and correlate the vector representation to a gesture input (e.g., a hand-wave, a pinch, a clap, a pen stroke, etc.) that has multiple dimensions. In other words, PSD 212 can detect a multi-dimension gesture without requiring the user to gesture at or near a screen or surface at which PSD 212 outputs information for display. Instead, PSD 212 can detect a multi-dimensional gesture performed at or near a sensor which may or may not be located near the screen or surface at which PSD 212 outputs information for display.

One or more processors 240 may implement functionality and/or execute instructions within computing device 212. For example, processors 240 on computing device 212 may receive and execute instructions stored by storage components 248 that execute the functionality of modules 220, 222, 224, and 272. The instructions executed by processors 240 may cause computing device 210 to store information within storage components 248 during program execution. Examples of processors 240 include application processors, display controllers, sensor hubs, and any other hardware configure to function as a processing unit. Processors 240 may execute instructions of modules 220, 222, 224, and 272 to cause PSD 212 to render portions of content of display data as one of user interface screen shots 116 at PSD 212. That is, modules 220, 222, 224, and 272 may be operable by processors 240 to perform various actions or functions of computing device 210.

One or more storage components 248 within computing device 210 may store information for processing during operation of computing device 210 (e.g., computing device 210 may store data accessed by modules 220, 222, 224, and 272 during execution at computing device 210). In some examples, storage component 248 is a temporary memory, meaning that a primary purpose of storage component 248 is not long-term storage. Storage components 248 on computing device 220 may be configured for short-term storage of information as volatile memory and therefore not retain stored contents if powered off. Examples of volatile memories include random access memories (RAM), dynamic random access memories (DRAM), static random access memories (SRAM), and other forms of volatile memories known in the art.

Storage components 248, in some examples, also include one or more computer-readable storage media. Storage components 248 may be configured to store larger amounts of information than volatile memory. Storage components 248 may further be configured for long-term storage of information as non-volatile memory space and retain information after power on/off cycles. Examples of non-volatile memories include magnetic hard discs, optical discs, floppy discs, flash memories, or forms of electrically programmable memories (EPROM) or electrically erasable and programmable (EEPROM) memories. Storage components 248 may store program instructions and/or information (e.g., data) associated with modules 220, 222, 224, and 272, as well as data stores 280.

Application modules 224 represent all the various individual applications and services executing at computing device 210. A user of computing device 210 may interact with an interface (e.g., a graphical user interface) associated with one or more application modules 224 to cause computing device 210 to perform a function. Numerous examples of application modules 224 may exist and include, a calendar application, a personal assistant or prediction engine, a search application, a map or navigation application, a transportation service application (e.g., a bus or train tracking application), a social media application, a game application, an e-mail application, a messaging application, an Internet browser application, or any and all other applications that may execute at computing device 210.

Application modules 224 may store application information at application information data store 280D for later retrieval and use in performing a function. For example, a calendar application of modules 224 may store an electronic calendar at data store 280D. Similarly, an e-mail application, messaging application, search application, transportation service application, or any other one of application modules 224 may store information or data for later retrieval at data store 280D.

With explicit permission from a user of computing device 210, modules 220, 222, and 272 may have access to information stored at data store 280D. For example, as described below, fitness module 210 may access data store 280D for calendar information, communication information, transportation information, or any other application information stored at data store 280D to determine a recommended exercise or appropriate time to recommend an exercise to a user of computing device 210. Said differently, fitness module 210 may use the application data (also referred to as "application information") stored at data store 280D as contextual information for determining a recommended physical activity for the user to perform and/or a current activity associated with the user. As such, contextual information data store 280B may include application information data store 280D as part of data store 280B or as a separate component, such that contextual information associated with computing device 210 includes sensor data obtained from one or more sensors 214, application data obtained from one or more application module 224 executing at computing device 210, calendar information associated with the user of computing device 210, and any all other information obtained by computing device 210 that can assist fitness module 220 in recommending exercises to a user.

Activity tracking module 272 may determine, based on contextual information, one or more activities being performed by a user at a particular time. Activity tracking module 272 may perform similar operations as activity tracking module 164. In other words, activity tracking module 272 may execute a rules based algorithm or a machine learning system that predicts what a user of computing device 210 is doing at a given time. The rules based algorithm or a machine learning system may be based on various observations about user behavior for different contexts such that activity tracking module 272 outputs an indication (e.g., data) of the predicted activity for use by fitness module 220 in recommending exercise(s).

In other examples, activity tracking module 272 represents an Application Programming Interface (API) associated with activity tracking module 164 of information server system 160. Activity tracking module 272 may provide an interface for receiving input to activity tracking module 164, and providing output received from activity tracking module 164, to other modules, applications, and/or components executing at computing device 210. For example, activity tracking module 272 may receive, as input from fitness module 220, an identifier of computing device 210 and/or contextual information associated with computing device 210 and in response, query activity tracking module 164 for an indication of one or more activities likely being performed by a user of computing device 210 at a particular time. Activity tracking module 272 may output the indication of the one or more activities back to fitness module 220 for inferring one or more recommended exercises.

Fitness module 220 may provide similar functionality as fitness module 120, of computing device 110, shown in FIG. 1. That is, fitness management module 220 may determine, based on contextual information stored at data store 280B, whether a user of computing device 210 has been physically inactive for a prolonged period of time and if so, determine a recommended physical activity the user could perform at the current time to become more physically active.

Fitness module 220 includes suggestion module 222 for determining the recommended physical activity the user could perform at the current time. In addition, suggestion module 222 may determine when fitness module 220 should refrain from recommending physical activity (e.g., when a user may have difficulty exercising while also concurrently performing a particular activity at a particular time). For example, suggestion module 222 may execute as a rules based algorithm or function as a machine learning system that predicts, using contextual information, what types of exercises that a user of computing device 210 could perform to become more physically active, at a particular time. The rules based algorithm or a machine learning system that computing device 210 stores at exercise rules data store 280C.

Exercise rules data store 280C may be based on various observations about user behavior for different contexts. Some of the rules stored at data store 280C may provide suggestion module 222 with an indication of one or more recommended physical activities that a user could perform for a particular context. Whereas other rules stored at data store 280C may provide suggestion module 222 with an indication of whether the one or more recommended physical activities are compatible with a particular activity being performed by the user for the particular context.

For example, suggestion module 222 may provide contextual information, fitness information, and/or application information as input to exercise rules data store 280C and in response, receive an indication of one or more recommended physical activities that the user could perform for the particular context. The one or more recommended physical activities may be ranked with a "score" that suggestion module 222 uses to discern which of the one or more physical activities to recommend. For instance, suggestion module 222 may refrain from recommending a physical activity if the associated score that the rules outputs does not satisfy a threshold (e.g., ninety percent, etc.).

In some examples, suggestion module 222 performs similar operations as device context module 162 for determining a user context of a user of computing device 210 based on contextual information (e.g., fitness information, application information, communication information, location information, sensor information, and all other information associated with a user of computing device 210) stored at data stores 280A, 280B, and 280D. Suggestion module 222 may determine a user context and provide the user context as input to the one or more rules suggestion module 222 maintains for determine a recommended exercise or a recommended physical activity. Suggestion module 222 may provide the user context as input to activity tracking module 272 for determining a current physical activity or other current activity being performed by a user of computing device 210, at a particular time.

In operation, responsive to determining that movement associated with computing device 210 does not satisfy an activity threshold indicative of a user of the computing device being in a physically active state, suggestion module 222 may determine a recommended physical activity for the user to perform. For instance, suggestion module 222 may receive sensor information from one or more sensor components 214. In some examples, suggestion module 222 may receive accelerometer readings or an output from sensor components 214 derived from the accelerometer readings, indicative of movement associated with computing device 210.

Suggestion module 222 may maintain a timer that counts or otherwise determines an amount of time that tracks a duration of time between "large" movements associated with computing device 210 (e.g., movements that exceed an activity threshold). For example, large movements may correspond to certain levels of acceleration that suggestion module 222 typically observes when a user stands up from a seated or lying position, begins walking, jogging, riding a bicycle, or otherwise transitioning into performing some other non-sedentary activity. Suggestion module 222 may determine that an alert to become more active may be appropriate in cases when the amount of time between large movements exceeds a time threshold (e.g., one half hour, one hour, etc.).

Responsive to determining that the movement associated with computing device 210 satisfies an activity threshold indicative of a user of the computing device being in a non-physically active state or a sedentary state, suggestion module 222 may determine a recommended physical activity for the user to perform. In some examples, suggestion module 222 may determine the recommended physical activity based on contextual information, a fitness goal associated with the user, historical activity information associated with the user, a current activity associated with the user, and/or any and all other information associated with the user.

For example, fitness module 220 may maintain fitness information about a user of computing device 210 at fitness information data store 280A. Suggestion module 222 may perform a lookup of the information contained at data store 280A to determine a fitness goal associated with the user. The fitness goal may be a goal that the user has to walk a certain quantity of steps each day. Suggestion module 222 may determine, based on the goal, that a recommended exercise or a recommended physical activity for the user to perform is walking.

Fitness module 220 may maintain historical activity information associated with the user of computing device 210 at fitness information data store 280A. Suggestion module 222 may perform a lookup of the historical information contained at data store 280A to determine a fitness goal associated with the user. The historical information may include information about an average quantity of steps that the user typically takes for a particular day. Suggestion module 222 may determine, based on the historical information, that a recommended physical activity for the user to perform is walking additional steps in order to maintain pace with the typical quantity of steps or otherwise typical level of fitness that the user maintains in any given particular day.

To determine whether the current time is an appropriate time to nudge the user to become active by, for example, outputting an alert as a notification of the recommended physical activity, suggestion module 222 may determine the current activity being performed by the user and then determine whether the current activity supports performing the recommended physical activity. In some examples, suggestion module 222 may determine the current activity by sending at least a portion of the contextual information associated with the computing device to a remote activity tracking system, querying the remote activity tracking system for an indication of the current activity, and receiving, from the remote activity tracking system, the indication of the current activity. For instance, suggestion module 222 may call an API provided by activity tracking module 272 by providing contextual information and/or a device identifier associated with computing device 220 (e.g., an account name, a phone number, an e-mail address, or some other identifying information) as inputs to the API and in response, receive an indication of the current activity. The indication of the current activity may include data that indicates the user of computing device 220 is attending a work meeting at a current location.

In some examples, suggestion module 222 may determine the recommended physical activity based at least in part on the current activity. For example, if suggestion module 222 infers a low amount of activity by the user of computing device 210 during a thirty minute meeting or car ride, suggestion module 222 may determine different recommended physical activities than if the low amount of activity is detected during a sixty or ninety minute meeting or car ride. For instance, suggestion module 222 may determine that simply standing for a minute or two is an appropriate recommended physical activity during a thirty minute meeting whereas breaking and taking a five minute break to walk to the bathroom and back may be an appropriate recommended physical activity during a sixty or ninety minute meeting.

Suggestion module 222 may determine whether a degree of likelihood that the recommended physical activity can be performed concurrently with the current activity satisfies a probability threshold. For example, suggestion module 222 may provide the recommended physical activity (e.g., walking) and the current activity (e.g., attending a work meeting) as inputs into an exercise rule stored at data store 280C and in response receive a score, a probability, or other degree of likelihood indicative of whether the recommended physical activity and the current activity can be performed concurrently. Put another way, suggestion module 222 may use the exercise rules stored at data store 280C to determine with a degree of certainty whether the user of computing device 210 can go for a walk and attend a work meeting at a current location, at the same time.

Responsive to determining that a degree of likelihood that the recommended physical activity can be performed concurrently with the current activity satisfies a probability threshold, suggestion module 222 may cause fitness module 220 to output a notification of the recommended physical activity (e.g., as a graphical notification at PSD 212 and/or as an audible or other type of alert using output components 246). For instance, suggestion module 222 may utilize a probability threshold (e.g. fifty percent, eighty percent, etc.).

If the recommended physical activity and current activity can be performed with a likelihood that satisfies the probability threshold, suggestion module 222 may cause fitness module 220 to output a notification. Else, if the recommended physical activity and current activity can be performed with a likelihood that does not satisfy the probability threshold, suggestion module 222 may cause fitness module 220 to refrain from outputting the notification.

For instance, suggestion module 222 may determine that in cases when a user of computing device 220 is attending an in-person meeting at a work location at which other meeting participants are attending, that the user is only able to stand up and begin walking around approximately twenty percent of the time. In this example, suggestion module 222 may cause fitness module 220 to refrain from outputting the notification.

In some examples, suggestion module 222 may defer output of the notification until a later time. Suggestion module 222 may determine that after the meeting, the user can stand up and begin walking and cause fitness module 220 to output the notification at that later time such that the notification is delivered to the user at a time that is more appropriate or otherwise, more likely to illicit a response and get the user to perform the recommended exercise.

In the case where suggestion module 222 outputs a deferred notification (e.g., a notification that was deferred until a later time), suggestion module 222 may output the deferred notification with an increase in graphical, tactile, and/or audible intensity such that the notification is more apparent to the user. In other words, after suggestion module 222 determines that the user will not become active at a particular time, suggestion module 222 may ramp up the alerting to increase the likelihood that the user will respond to the notification by performing the recommended exercise at a later time.

In some examples, suggestion module 222 may anticipate that a user may be unable to perform recommended exercises at a future time and pre-emptively output notifications of recommended physical activities at earlier times. For instance, suggestion module 222 may determine that a future meeting will last for a half hour or more and cause fitness module 220 to output the notification ten minutes before the meeting as a suggestion or nudge that the user begin walking to the meeting earlier, to get some walking in before the meeting.

However, for some meetings (e.g., virtual meetings or teleconference calls at which the user is attending from his or her office), suggestion module 222 may determine that the user is able to stand up and begin walking approximately ninety percent of the time. In this example, suggestion module 222 may cause fitness module 220 to output the notification.

In some examples, suggestion module 222 may determine, based at least in part on the contextual information, a future time at which the user of the computing device will be unable to perform the recommended physical activity. Suggestion module 222 may cause fitness module to output the notification of the recommended physical activity in response to determining that the recommended physical activity can be performed before the future time.

For example, in addition to determining whether the recommended physical activity and the current activity can be performed concurrently, suggestion module 222 may determine whether the user can finish performing the recommended physical activity without interfering with the user's future schedule. For example, if the user is between work meetings, suggestion module 222 may determine (e.g., based on calendar information stored at application information data store 280D) that the user has fifteen minutes before the start of a subsequent meeting. Suggestion module 222 may cause fitness module 220 to output a graphical notification at PSD 212 suggesting that the user go for a ten minute walk so as to get in some extra exercise without being late for the subsequent meeting.

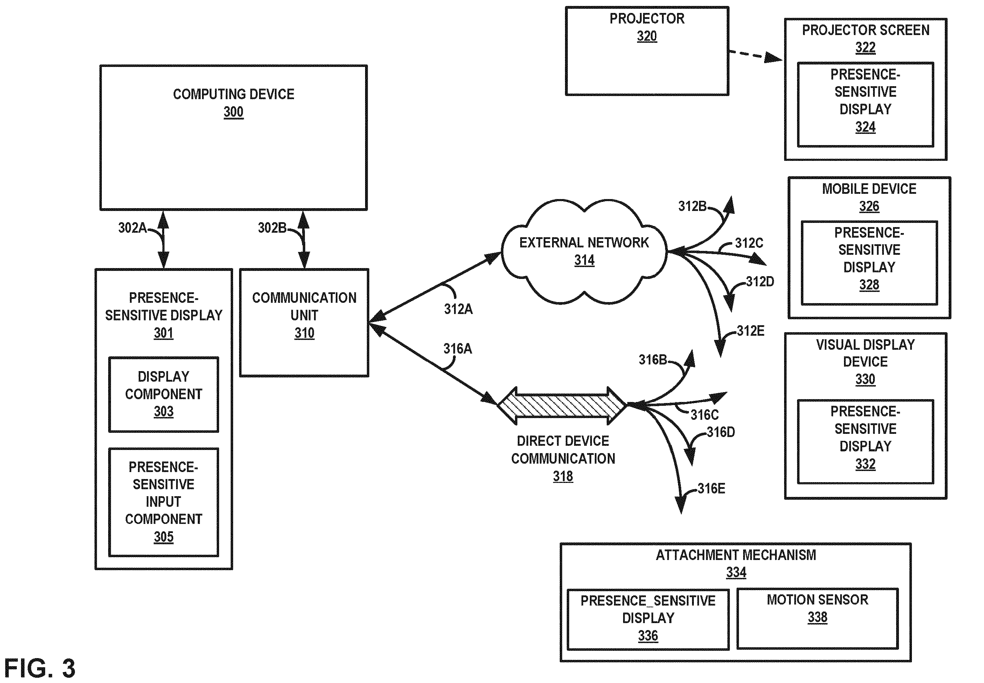

FIG. 3 is a block diagram illustrating computing device 300 as an example computing device that outputs graphical content for display at a remote device, in accordance with one or more techniques of the present disclosure. Computing device 300 is an additional example of computing device 110 of FIG. 1 and computing device 210 of FIG. 2.

Graphical content, generally, may include any visual information that may be output for display, such as text, images, a group of moving images, etc. The example shown in FIG. 3 includes a computing device 300, presence-sensitive display 301, communication unit 310, projector 320, projector screen 122, mobile device 326, visual display device 330, and attachment mechanism 334 (e.g., a component of a wearable computing device that attaches to a user's body or clothing). Although shown for purposes of example in FIGS. 1 and 2 as stand-alone computing devices 110 and 210, respectively, a computing device such as computing device 300 may, generally, be any system, device, or component thereof that includes a processor or other suitable computing environment for executing software instructions and, for example, need not include a presence-sensitive display.

As shown in the example of FIG. 3, computing device 300 may be a processor that includes functionality as described above with respect to processors 240 in FIG. 2. In such examples, computing device 300 may be operatively coupled to presence-sensitive display 301 by a communication channel 302A, which may be a system bus or other suitable connection. Computing device 300 may also be operatively coupled to communication unit 310, further described below, by a communication channel 302B, which may also be a system bus or other suitable connection. Although shown separately as an example in FIG. 3, computing device 300 may be operatively coupled to presence-sensitive display 301 and communication unit 310 by any number of one or more communication channels.

Presence-sensitive display 301 may include display device 303 and presence-sensitive input device 305. Display device 303 may, for example, receive data from computing device 300 and display the graphical content. In some examples, presence-sensitive input device 305 may determine one or more user inputs (e.g., continuous gestures, multi-touch gestures, single-touch gestures, etc.) at presence-sensitive display 301 using capacitive, inductive, and/or optical recognition techniques and send indications of such user input to computing device 300 using communication channel 302A. In some examples, presence-sensitive input device 305 may be physically positioned on top of display device 303 such that, when a user positions an input unit over a graphical element displayed by display device 303, the location at which presence-sensitive input device 305 corresponds to the location of display device 303 at which the graphical element is displayed. In other examples, presence-sensitive input device 305 may be positioned physically apart from display device 303, and locations of presence-sensitive input device 305 may correspond to locations of display device 303, such that input can be made at presence-sensitive input device 305 for interacting with graphical elements displayed at corresponding locations of display device 303.

As shown in FIG. 3, computing device 300 may also include and/or be operatively coupled with communication unit 310. Communication unit 310 may include functionality of communication unit 244 as described in FIG. 2. Examples of communication unit 310 may include a network interface card, an Ethernet card, an optical transceiver, a radio frequency transceiver, or any other type of device that can send and receive information. Other examples of such communication units may include Bluetooth ^{®}, 3G, and Wi-Fi ^{®} radios, Universal Serial Bus (USB) interfaces, etc. Computing device 300 may also include and/or be operatively coupled with one or more other devices, e.g., input devices, output devices, memory, storage devices, etc. that are not shown in FIG. 6 for purposes of brevity and illustration.

FIG. 3 also illustrates a projector 320 and projector screen 322. Other examples of projection devices may include electronic whiteboards, holographic display devices, and any other suitable devices for displaying graphical content. Projector 320 and projector screen 322 may include one or more communication units that enable the respective devices to communicate with computing device 300. In some examples, the one or more communication units may enable communication between projector 320 and projector screen 322. Projector 320 may receive data from computing device 300 that includes graphical content. Projector 320, in response to receiving the data, may project the graphical content onto projector screen 322. In some examples, projector 320 may determine one or more user inputs (e.g., continuous gestures, multi-touch gestures, single-touch gestures, double-bezel gestures, etc.) at projector screen using optical recognition or other suitable techniques and send indications of such user input using one or more communication units to computing device 300. In such examples, projector screen 322 may be unnecessary, and projector 320 may project graphical content on any suitable medium and detect one or more user inputs using optical recognition or other such suitable techniques.

Projector screen 322, in some examples, may include a presence-sensitive display 324. Presence-sensitive display 324 may include a subset of functionality or all of the functionality of PSDs 112 and 212 as described in this disclosure. In some examples, presence-sensitive display 324 may include additional functionality. Projector screen 322 (e.g., an electronic whiteboard), may receive data from computing device 300 and display the graphical content. In some examples, presence-sensitive display 324 may determine one or more user inputs (e.g., continuous gestures, multi-touch gestures, single-touch gestures, double-bezel gestures, etc.) at projector screen 322 using capacitive, inductive, and/or optical recognition techniques and send indications of such user input using one or more communication units to computing device 300.

FIG. 3 also illustrates mobile device 326 and visual display device 330. Mobile device 326 and visual display device 330 may each include computing and connectivity capabilities. Examples of mobile device 326 may include e-reader devices, convertible notebook devices, hybrid slate devices, etc. Examples of visual display device 330 may include other semi-stationary devices such as televisions, computer monitors, etc. As shown in FIG. 3, mobile device 326 may include a presence-sensitive display 328. Visual display device 330 may include a presence-sensitive display 332. Presence-sensitive display 332, for example, may receive data from computing device 300 and display the graphical content. In some examples, presence-sensitive display 332 may determine one or more user inputs (e.g., continuous gestures, multi-touch gestures, single-touch gestures, double-bezel gestures, etc.) at projector screen using capacitive, inductive, and/or optical recognition techniques and send indications of such user input using one or more communication units to computing device 300.

As described above, in some examples, computing device 300 may output graphical content for display at presence-sensitive display 301, which is coupled to computing device 300 by a system bus or other suitable communication channel. Computing device 300 may also output graphical content for display at one or more remote devices, such as projector 320, projector screen 322, mobile device 326, and visual display device 330. For instance, computing device300 may execute one or more instructions to generate and/or modify graphical content in accordance with techniques of the present disclosure. Computing device 300 may output the data that includes the graphical content to a communication unit of computing device 300, such as communication unit 310. Communication unit 310 may send the data to one or more of the remote devices, such as projector 320, projector screen 322, mobile device 326, and/or visual display device 330. In this way, computing device 300 may output the graphical content for display at one or more of the remote devices. In some examples, one or more of the remote devices may output the graphical content at a display device, such as a presence-sensitive display, that is included in and/or operatively coupled to the respective remote device.

In some examples, computing device 300 may not output graphical content at presence-sensitive display 301 that is operatively coupled to computing device 300. In other examples, computing device 300 may output graphical content for display at both a presence-sensitive display 301 that is coupled to computing device 300 by communication channel 302A, and at a display of one or more the remote devices. In such examples, the graphical content may be displayed substantially contemporaneously at each respective device. For instance, some delay may be introduced by the communication latency to send the data that includes the graphical content to the remote device. In some examples, graphical content generated by computing device 300 and output for display at presence-sensitive display 301 may be different than graphical content display output for display at one or more remote devices.

Computing device 300 may send and receive data using any suitable communication techniques. For example, computing device 300 may be operatively coupled to external network 314 using network link 312A. Each of the remote devices illustrated in FIG. 3 may be operatively coupled to network external network 314 by one of respective network links 312B, 312C, 312D, and 312E. External network 314 may include network hubs, network switches, network routers, etc., that are operatively inter-coupled thereby providing for the exchange of information between computing device 300 and the remote devices illustrated in FIG. 3. In some examples, network links 312A-312E may be Ethernet, ATM or other network connections. Such connections may be wireless and/or wired connections.

In some examples, computing device 300 may be operatively coupled to one or more of the remote devices included in FIG. 3 using direct device communication 318. Direct device communication 318 may include communications through which computing device 300 sends and receives data directly with a remote device, using wired or wireless communication. That is, in some examples of direct device communication 318, data sent by computing device 300 may not be forwarded by one or more additional devices before being received at the remote device, and vice-versa. Examples of direct device communication 318 may include Bluetooth^{®}, Near-Field Communication, Universal Serial Bus, infrared, etc. One or more of the remote devices illustrated in FIG. 3 may be operatively coupled with computing device 300 by communication links 316A-316E. In some examples, communication links 316A-316E may be connections using Bluetooth^{®}, Near-Field Communication, Universal Serial Bus, infrared, etc. Such connections may be wireless and/or wired connections.

In accordance with techniques of the disclosure, computing device 300 can be operable to output a graphical alert at any one or more of presence-sensitive displays 324, 328, 332, and/or 336 as a notification of a recommended physical activity for a user of computing device 300 to perform. In some examples, computing device 100 may output the notification of the recommended physical activity by outputting the notification for subsequent output at attachment mechanism 334 (e.g., a wearable device). For instance, computing device 300 may output the graphical alert, the tactile alert, and/or the audible alert through external network at any one or more of projector 320, screen 322, device 326, device 330 and/or attachment mechanism 334. In some examples, attachment mechanism 334 may output the graphical notification at presence-sensitive display 336 in addition to, or as opposed to, outputting the graphical notification at presence-sensitive display 328 of mobile device 326.

In some examples, if a user begins moving and computing device 300 determines that the user is performing the recommended physical activity, computing device 300 may further output a subsequent notification congratulating the user. For example, the subsequent notification may congratulate the user in moving or completing the exercise.

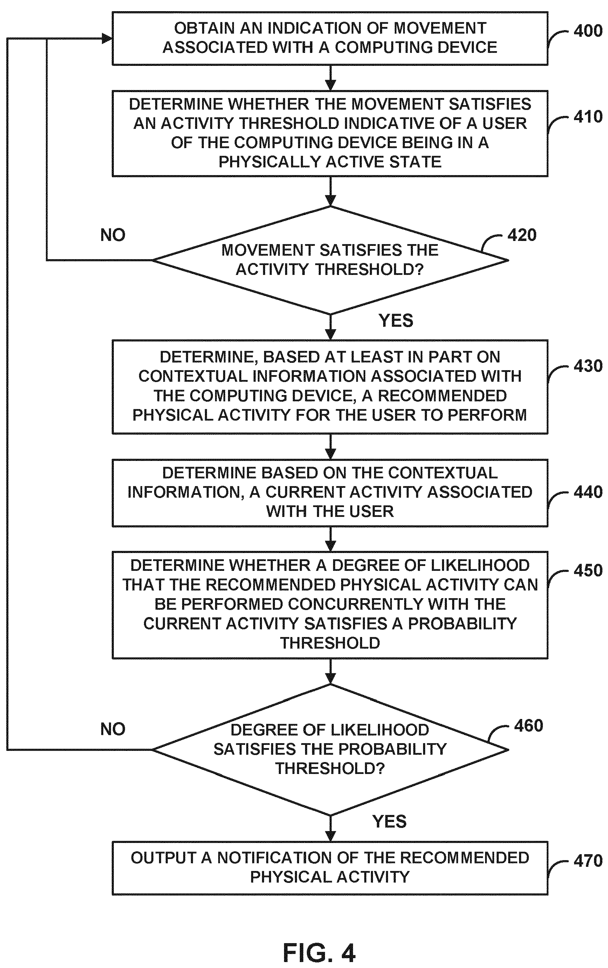

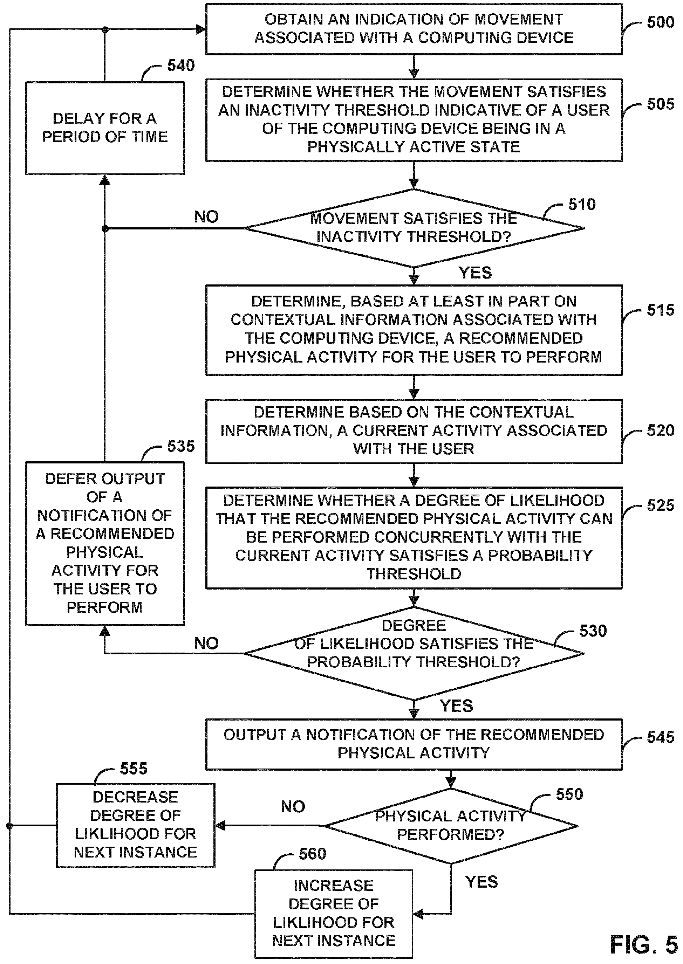

FIGS. 4 and 5 are flowcharts illustrating example operations of an example computing device configured to recommend physical activities for a user of the computing device to perform, at appropriate times, in accordance with one or more aspects of the present disclosure. The processes of FIGS. 4 and FIG. 5 may each be performed by one or more processors of a computing device, such as computing device 110 of FIG. 1 and/or computing device 210 of FIG. 2. The steps of the processes FIGS. 4 and FIG. 5 may in some examples, be repeated, omitted, and/or performed in any order. For purposes of illustration, FIGS. 4 and 5 are described below within the context of computing device 210 of FIG. 2.

In the example of FIG. 4, computing device 210 may obtain (400) an indication of movement associated with the computing device. For example, fitness module 220 may occasionally receive accelerometer information from sensor components 214.

Computing device 210 may determine (410) whether the movement satisfies an activity threshold indicative of a user of the computing device being in a physically active state. For example, fitness module 220 may determine whether the accelerometer information indicates that the user has been mostly sedentary or somewhat active for a prolonged period of time (e.g., one half hour).

Responsive to determining (420) the movement does not satisfy (e.g., is less than or alternatively, is greater than or equal to) the activity threshold, computing device 210 may determine (430), based at least in part on contextual information associated with computing device 210, a recommended physical activity for the user to perform. For example, fitness module 220 may infer that the lack of movement indicates the user has been sitting for a prolonged period of time and determine based on exercise rules data store 280C that the user should stand or perform a suggested number of squats. Otherwise, responsive to determining (420) the movement satisfies the activity threshold, computing device 210 may forego recommending any further physical activity based on a determination by fitness module 220 that the movement information indicates that the user is already physically active.

In some examples, computing device 210 may obtain the contextual information used to determine the recommended physical activity from a remote system, such as information server system 160. For example, computing device 210 may send, to a remote system, sensor information obtained by one or more sensor components 214 and responsive to sending the sensor information to the remote system, receive, from the remote system, the contextual information associated with the computing device. The contextual information received from the remote system may be based at least in part on the sensor information. For example, the contextual information received from information server system 160 may include contextual information that reflects not only the sensor information (e.g., location, movement, speed, and the like) but also other data in the network (e.g., local weather).

In some examples, computing device 210 may determine the recommended physical activity for the user to perform by sending, by the computing device, to a remote system, at least a portion of the contextual information associated with the computing device, and receiving, by the computing device, from the remote system, the recommended physical activity. The recommended physical activity received from the remote system may be based at least in part on the context information associated with the computing device and may be further based at least in part on other information associated with the user. For example, computing device 210 may query information server system 160 for a recommended physical activity. Information server system 160 may determine a recommended physical activity that may be suitable for performing given a particular context, but also may be personalized for the user (e.g., based on health conditions of the user, fitness goals, and other information). For example information server system 160 may recommend a more strenuous physical activity for a particular context if the user is determined to be generally in good health and may recommend a less strenuous physical activity for the particular context if the user is determined to have high blood pressure, is overweight, or has some other health condition.

Computing device 210 may determine (440), based on the contextual information, a current activity associated with the user. For example, fitness module 220 may determine based on application data stored at data store 280D or other information received from application modules 224 that the user is playing an electronic game executing at computing device 210 at the current time.

Computing device 210 may determine (450) whether a degree of likelihood that the recommended physical activity can be performed concurrently with the current activity satisfies a probability threshold. For example, using one or more exercise rules at data store 280C, fitness module 220 may determine that the game does not require extreme focus and/or that a user can likely stand up while playing the electronic game without interfering with the game play.

Responsive to determining (460) that the degree of likelihood satisfies the probability threshold, computing device 210 may output (470) a notification of the recommended physical activity. For example, fitness module 220 may cause PSD 212 to present a graphical alert as a notification that the user should try standing or squatting in place for a minute or two while playing the game to become more active.

In this way, computing device 210 may output the notification of the recommended physical activity in response to determining that the notification and/or physical activity will not interfere with a current activity being performed by the user. Conversely, computing device 210 may defer output of the notification until a later time in response to determining that the notification and/or physical activity may interfere with a current activity being performed by the user.

For instance, consider the example of FIG. 5. Computing device 210 may obtain (500) an indication of movement associated with computing device 210. For example, fitness module 220 may receive sensor information (e.g., continuous accelerometer movement coupled with large / fast GPS location changes) from sensor components 214.

Computing device 210 may determine (505) whether the movement satisfies an inactivity threshold indicative of a user of computing device 210 being in a physically active state. For example, fitness module 220 may compare the sensor information received from sensor components 214 to patterns of movement typically observed when a user of computing device 210 is sitting and not being particularly active.

Responsive to determining (510) that the movement satisfies the inactivity threshold, computing device 210 may forgo recommending any further physical activity and delay (540) for a period of time before performing the process of FIG. 5 again. For example, fitness module 220 may determine the sensor information received from sensor components 214 corresponds to patterns of movement typically observed when a user of computing device 210 is not sitting, but rather the user is being physically active.

Conversely, responsive to determining (510) that the movement does not satisfy the activity threshold, computing device 210 may determine (515), based on contextual information associated with computing device 210, a recommended physical activity for the user to perform. For example, based on the sensor information obtained from sensor components 214, fitness module 220 may infer that the sensor information corresponds to patterns of movement typically observed when a user of computing device 210 is sitting. Fitness module 220 may receive information from context module 162 of information server system 160 that the current context of computing device 210 corresponds to the inside of a moving vehicle (e.g., train, bus, car, other vehicle). Based on exercise rules data store 280C, fitness module 220 may determine one or more recommended exercises for the user to perform (e.g., stand, touch toes, stretch, perform a suggested number of squats, or perform some other physical activity) while inside the moving vehicle.

In some examples, fitness module 220 may query a driving application as one of application modules 124. The driving application may interface with an automobile system that provides contextual information back to the driving application, about the state of the automobile system. The driving application may provide fitness module 220 with application data (e.g., an indication) indicating that computing device 210 is synched up with, docked at, or otherwise inside an automobile vehicle.

Computing device 210 may determine (520) based on the contextual information, a current activity associated with the user. For example, fitness module 220 may provide the current context of computing device 210 to activity tracking module 272 and receive in response, data that indicates the activity that the user may be performing while inside the moving vehicle. For example, activity tracking module 272 may infer based on the context and other contextual information stored at data store 280B that the user is driving the moving vehicle.

Computing device 210 may determine (525) whether a degree of likelihood that the recommended physical activity can be performed concurrently with the current activity satisfies a probability threshold. For example, fitness module 220 may feed the current activity and recommended exercises as inputs into one or more exercise rules 280C for determining whether any of the recommended exercises can be performed while driving a moving vehicle. Fitness module 220 may receive as output from the one or more exercise rules 280C a probability, score, or other indication of whether each of the recommended exercises can be performed simultaneously with the current activity.

Responsive to determining that none of the exercises is compatible with the current activity (e.g., the exercises cannot be performed safely while driving a moving vehicle), computing device 210 may defer (535) output of a notification of a recommended physical activity for the user to perform and delay (540) for a period of time re-running the process of FIG. 5. For example, fitness module 220 may input the recommended physical activity into an activity queue that fitness module 220 may surface at a later time after re-running steps 500-525.

Conversely, responsive to determining that one or more of the exercises is compatible with the current activity computing device 210 may output (545) a notification of the recommended physical activity. For example, fitness module 220 may occasionally request updates from the driving application about the operating state of the automobile to determine whether the user is still driving the moving vehicle. Based on the updated information received from the driving application, fitness module 220 may, relying on activity tracking module 124, determine that computing device 210 is no longer synched up with, docked at, or otherwise inside an automobile vehicle and that the user is therefore not driving the moving vehicle. In response to determining that the user is no longer driving, fitness module 220 may cause PSD 212 to output a graphical indication of the notification (e.g., to remind the user to get some exercise in now that he or she is no longer driving).

In some examples, computing device 210 may determine (550) whether the recommended physical activity was or was not performed after outputting the notification and in response, update the models and rules that computing device 210 uses, accordingly. For example, fitness module 220 may analyze sensor information obtained from sensor components 214 after causing PSD 212 to output the notification. Responsive to determining the sensor information corresponds to patterns of movement typically observed when the user performs the recommended exercise, fitness module 220 may increase (560) the degree of likelihood that the machine learning system provides for the particular context. Conversely, responsive to determining the sensor information does not correspond to patterns of movement typically observed when the user performs the recommended exercise, fitness module 220 may decrease (555) the degree of likelihood that the machine learning system provides for the particular context.

In some examples, computing device 210 may update the probability thresholds as a way to update the models and rules. For example, fitness module 220 may analyze sensor information obtained from sensor components 214 after causing PSD 212 to output the notification. Responsive to determining the sensor information corresponds to patterns of movement typically observed when the user performs the recommended exercise, fitness module 220 may decrease the probability threshold the machine learning system provides for the particular context to make it more likely that the exercises will be recommended in the future for the particular context. Conversely, responsive to determining the sensor information does not correspond to patterns of movement typically observed when the user performs the recommended exercise, fitness module 220 may increase the probability threshold the machine learning system provides for the particular context to make it less likely that the exercises will be recommended in the future for the particular context.

By using context clues such as the user's current activity, calendar, and other contextual information, the techniques of this disclosure may enable a computing device to send or suppress notifications until the computing device is confident that the user is able to move around and perform a recommended physical activity. For instance, if a calendar associated with a user indicates that he or she is in a meeting, the user may ignore a notification to get up and walk around. Using the calendar, the device may determine when the meeting ends and, if there is a gap in the user's calendar between two events, the device may output a notification suggesting that the user take advantage of that time between events to get some additional exercise or movement.

When determining the language to use in a motivational notification, the computing device may process the user's previous fitness data for various time frames. By comparing a current day's activity to a previous day's activity, or activity from the same day during a previous week, a weekly average, or a monthly average, the device may infer a fitness goal of a user or otherwise determine how much activity to recommend that the user perform during successive notifications.

In one or more examples, the functions described may be implemented in hardware, software, firmware, or any combination thereof. If implemented in software, the functions may be stored on or transmitted over, as one or more instructions or code, a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media, which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

By way of example, and not limitation, such computer-readable storage media can comprise RAM, ROM, EEPROM, CD-ROM or other optical disk storage, magnetic disk storage, or other magnetic storage devices, flash memory, or any other medium that can be used to store desired program code in the form of instructions or data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transient media, but are instead directed to non-transient, tangible storage media. Disk and disc, as used herein, includes compact disc (CD), laser disc, optical disc, digital versatile disc (DVD), floppy disk and Blu-ray disc, where disks usually reproduce data magnetically, while discs reproduce data optically with lasers. Combinations of the above should also be included within the scope of computer-readable media.

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor," as used herein may refer to any of the foregoing structure or any other structure suitable for implementation of the techniques described herein. In addition, in some aspects, the functionality described herein may be provided within dedicated hardware and/or software modules. Also, the techniques could be fully implemented in one or more circuits or logic elements.

The techniques of this disclosure may be implemented in a wide variety of devices or apparatuses, including a wireless handset, an integrated circuit (IC) or a set of ICs (e.g., a chip set). Various components, modules, or units are described in this disclosure to emphasize functional aspects of devices configured to perform the disclosed techniques, but do not necessarily require realization by different hardware units. Rather, as described above, various units may be combined in a hardware unit or provided by a collection of interoperable hardware units, including one or more processors as described above, in conjunction with suitable software and/or firmware.

The scope of protection is defined by the claims.

## Claims

1. A method, comprising:
obtaining (400), by a computing device (110, 210), an indication of a movement associated with the computing device; and
responsive to determining (410, 420) that the movement does not satisfy an activity threshold indicative of a user of the computing device being in a physically active state:
determining (430), by the computing device, based at least in part on contextual information associated with the computing device, a recommended exercise for the user to perform, wherein the recommended exercise is further determined based at least in part on achieving a fitness goal associated with the user, wherein the fitness goal includes one or more of: a maximum heart rate level, a quantity of footsteps over a time duration, a distance traveled over a time duration, or an amount of time spent by the user performing exercise;
determining (440), by the computing device, at a current point in time and based at least in part on the contextual information, a current activity the user is likely performing at the current time, wherein the current activity is a sedentary or idle activity;
determining (450), based on the current activity and the recommended exercise, a degree of likelihood that the user can perform the recommended exercise and the current activity at the same time; and
responsive to determining (460) that the degree of likelihood satisfies a probability threshold, outputting (470), by the computing device, a notification of the recommended exercise.

2. The method of claim 1, wherein obtaining the indication of the movement associated with the computing device comprises obtaining sensor data from one or more sensors (114, 214) of the computing device.

3. The method of claim 2, further comprising:
after outputting the notification, analysing sensor data from the one or more sensors and determining whether the sensor data corresponds to patterns of movement typically observed when the user performs the recommended exercise; and
adjusting the probability threshold based on the determining.

4. The method of claim 3, wherein the adjusting comprises:
decreasing the probability threshold when the sensor data corresponds to patterns of movement typically observed when the user performs the recommended exercise; and
increasing the probability threshold when the sensor data does not correspond to patterns of movement typically observed when the user performs the recommended exercise.

5. The method of any of claims 1-4, wherein the degree of likelihood is further determined based on one or more stored exercise rules.

6. The method of any of claims 1-5, wherein the recommended exercise is further determined based at least in part on historical activity information associated with the user and/or the current activity.

7. The method of any of claims 1-6, further comprising:
responsive to determining (460) that the degree of likelihood does not satisfy the probability threshold, refraining from outputting, by the computing device, the notification of the recommended exercise; and
after refraining from outputting the notification, re-running the method of claim 1 after a predetermined time period.

8. The method of any of claims 1-7, wherein:
the current activity comprises: driving or riding in a moving vehicle, sitting or standing at a desk, sitting in a meeting or lecture, standing at or near a bus stop, or playing an electronic game executing at the computing device; and/or
the recommended exercise comprises: standing, touching toes,
stretching, performing a suggested number of squats, or walking.

9. The method of any of claims 1-8, wherein the contextual information associated with the computing device comprises at least one of: sensor data obtained from one or more sensors (114, 214) of the computing device; application data obtained from one or more applications executing at the computing device, or calendar information associated with the user of the computing device (210).

10. The method of any of claims 1-9, wherein determining the current activity comprises:
sending, by the computing device, to a remote activity tracking system, at least a portion of the contextual information associated with the computing device;
querying, by the computing device, the remote activity tracking system for an indication of the current activity; and
receiving, by the computing device, from the remote activity tracking system, the indication of the current activity.

11. The method of any of claims 1-10, wherein outputting the notification of the recommended exercise comprises outputting, by the computing device, the notification for subsequent output at a wearable device,
wherein the notification comprises at least one of a graphical alert, a tactile alert, or an audible alert.

12. A computing device (110, 210) comprising:
at least one sensor component (114, 214) configured to obtain (400) sensor information indicative of a movement associated with the computing device;
at least one processor; and
at least one module operable by the at least one processor to perform the method of any of claims 1-11.

13. A system comprising means for performing the method of any of claims 1-11.

14. A non-transitory computer-readable medium comprising instructions that, when executed by one or more hardware processors, cause it or them to perform the method of any of claims 1-11.

15. A computer-program comprising instructions that, when executed by one or more hardware processors, cause the one or more hardware processors to perform the method of any of claims 1-11.

## Patentansprüche

1. Verfahren, umfassend:
Erhalten (400), durch eine Rechenvorrichtung (110, 210), einer Angabe einer Bewegung, die der Rechenvorrichtung zugeordnet ist; und
als Reaktion auf ein Bestimmen (410, 420), dass die Bewegung einen Aktivitätsschwellenwert nicht erfüllt, der angibt, dass sich ein Benutzer der Rechenvorrichtung in einem körperlich aktiven Zustand befindet:
Bestimmen (430), durch die Rechenvorrichtung, zumindest teilweise basierend auf Kontextinformationen, die der Rechenvorrichtung zugeordnet sind, einer empfohlenen Übung, die der Benutzer ausführen soll, wobei die empfohlene Übung ferner zumindest teilweise basierend auf einem Erreichen eines dem Benutzer zugeordneten Fitnessziels bestimmt wird, wobei das Fitnessziel eines oder mehrere der Folgenden beinhaltet:
ein maximales Herzfrequenzniveau, eine Anzahl von Schritten über eine Zeitdauer, eine über eine Zeitdauer zurückgelegte Strecke oder eine Zeitspanne, die der Benutzer mit einem Ausführen von Übungen verbringt;
Bestimmen (440), durch die Rechenvorrichtung, zu einem aktuellen Zeitpunkt und zumindest teilweise basierend auf den Kontextinformationen, einer aktuellen Aktivität, die der Benutzer wahrscheinlich zur aktuellen Zeit ausführt, wobei die aktuelle Aktivität eine sitzende oder inaktive Aktivität ist;
Bestimmen (450), basierend auf der aktuellen Aktivität und der empfohlenen Übung, eines Wahrscheinlichkeitsgrades, dass der Benutzer die empfohlene Übung und die aktuelle Aktivität gleichzeitig ausführen kann; und
als Reaktion auf ein Bestimmen (460), dass der Wahrscheinlichkeitsgrad einen Wahrscheinlichkeitsschwellenwert erfüllt, Ausgeben (470), durch die Rechenvorrichtung, einer Benachrichtigung über die empfohlene Übung.

2. Verfahren nach Anspruch 1, wobei Erhalten der Angabe der Bewegung, die der Rechenvorrichtung zugeordnet ist, Erhalten von Sensordaten von einem oder mehreren Sensoren (114, 214) der Rechenvorrichtung umfasst.

3. Verfahren nach Anspruch 2, ferner umfassend:
nach Ausgeben der Benachrichtigung Analysieren von Sensordaten von dem einen oder den mehreren Sensoren und Bestimmen, ob die Sensordaten Bewegungsmustern entsprechen, die typischerweise beobachtet werden, wenn der Benutzer die empfohlene Übung ausführt; und
Anpassen des Wahrscheinlichkeitsschwellenwerts basierend auf dem Bestimmen.

4. Verfahren nach Anspruch 3, wobei das Anpassen Folgendes umfasst:
Verringern des Wahrscheinlichkeitsschwellenwerts, wenn die Sensordaten Bewegungsmustern entsprechen, die typischerweise beobachtet werden, wenn der Benutzer die empfohlene Übung ausführt; und
Erhöhen des Wahrscheinlichkeitsschwellenwerts, wenn die Sensordaten keinen Bewegungsmustern entsprechen, die typischerweise beobachtet werden, wenn der Benutzer die empfohlene Übung ausführt.

5. Verfahren nach einem der Ansprüche 1-4, wobei der Wahrscheinlichkeitsgrad ferner basierend auf einer oder mehreren gespeicherten Übungsregeln bestimmt wird.

6. Verfahren nach einem der Ansprüche 1-5, wobei die empfohlene Übung ferner zumindest teilweise basierend auf historischen Aktivitätsinformationen bestimmt wird, die dem Benutzer und/oder der aktuellen Aktivität zugeordnet sind.

7. Verfahren nach einem der Ansprüche 1-6, ferner umfassend:
als Reaktion auf ein Bestimmen (460), dass der Wahrscheinlichkeitsgrad den Wahrscheinlichkeitsschwellenwert nicht erfüllt, Unterlassen eines Ausgebens, durch die Rechenvorrichtung, der Benachrichtigung über die empfohlene Übung; und
nach Unterlassen des Ausgebens der Benachrichtigung erneutes Ausführen des Verfahrens nach Anspruch 1 nach Ablauf einer vorbestimmten Zeitspanne.

8. Verfahren nach einem der Ansprüche 1 bis 7, wobei:
die aktuelle Aktivität Folgendes umfasst: Fahren oder Mitfahren in einem fahrenden Fahrzeug, Sitzen oder Stehen an einem Schreibtisch, Sitzen in einer Besprechung oder Vorlesung, Stehen an oder in der Nähe einer Bushaltestelle oder Spielen eines elektronischen Spiels, das auf der Rechenvorrichtung ausgeführt wird; und/oder
die empfohlene Übung Folgendes umfasst: Stehen, Berühren der Zehen, Dehnen, Ausführen einer vorgeschlagenen Anzahl von Kniebeugen oder Gehen.

9. Verfahren nach einem der Ansprüche 1-8, wobei die Kontextinformationen, die der Rechenvorrichtung zugeordnet sind, mindestens eines der Folgenden umfassen: Sensordaten, die von einem oder mehreren Sensoren (114, 214) der Rechenvorrichtung erhalten werden; Anwendungsdaten, die von einer oder mehreren Anwendungen erhalten werden, die auf der Rechenvorrichtung ausgeführt werden, oder Kalenderinformationen, die dem Benutzer der Rechenvorrichtung (210) zugeordnet sind.

10. Verfahren nach einem der Ansprüche 1-9, wobei Bestimmen der aktuellen Aktivität Folgendes umfasst:
Senden, durch die Rechenvorrichtung, mindestens eines Teils der Kontextinformationen, die der Rechenvorrichtung zugeordnet sind, an ein entferntes Aktivitätsverfolgungssystem;
Abfragen, durch die Rechenvorrichtung, des entfernten Aktivitätsverfolgungssystems nach einer Angabe der aktuellen Aktivität; und
Empfangen, durch die Rechenvorrichtung, von dem entfernten Aktivitätsverfolgungssystem, der Angabe der aktuellen Aktivität.

11. Verfahren nach einem der Ansprüche 1-10, wobei Ausgeben der Benachrichtigung über die empfohlene Übung Ausgeben, durch die Rechenvorrichtung, der Benachrichtigung zur anschließenden Ausgabe an einer tragbaren Vorrichtung umfasst,
wobei die Benachrichtigung mindestens einen von einem grafischen Alarm, einem taktilen Alarm oder einem hörbaren Alarm umfasst.

12. Rechenvorrichtung (110, 210), umfassend:
mindestens eine Sensorkomponente (114, 214), die konfiguriert ist, um Sensorinformationen zu erhalten (400), die eine Bewegung angeben, die der Rechenvorrichtung zugeordnet ist;
mindestens einen Prozessor; und
mindestens ein Modul, das durch den mindestens einen Prozessor betreibbar ist, um das Verfahren nach einem der Ansprüche 1-11 durchzuführen.

13. System, umfassend Mittel zum Durchführen des Verfahrens nach einem der Ansprüche 1-11.

14. Nichttransitorisches computerlesbares Medium, umfassend Anweisungen, die, wenn sie von einem oder mehreren Hardwareprozessoren ausgeführt werden, den einen oder die mehreren Hardwareprozessoren veranlassen, das Verfahren nach einem der Ansprüche 1-11 durchzuführen.

15. Computerprogramm, umfassend Anweisungen, die, wenn sie von einem oder mehreren Hardwareprozessoren ausgeführt werden, den einen oder die mehreren Hardwareprozessoren veranlassen, das Verfahren nach einem der Ansprüche 1-11 durchzuführen.

## Revendications

1. Procédé, comprenant :
l'obtention (400), par un dispositif informatique (110, 210), d'une indication d'un mouvement associé au dispositif informatique ; et
en réponse à la détermination (410, 420) du fait que le mouvement ne satisfait pas à un seuil d'activité indiquant qu'un utilisateur du dispositif informatique est dans un état physiquement actif :
la détermination (430), par le dispositif informatique, sur la base au moins en partie d'informations contextuelles associées au dispositif informatique, d'un exercice recommandé que l'utilisateur doit effectuer, dans lequel l'exercice recommandé est en outre déterminé sur la base au moins en partie de la réalisation d'un objectif de forme physique associé à l'utilisateur, dans lequel l'objectif de forme physique comporte l'un ou plusieurs parmi : un niveau de fréquence cardiaque maximal, une quantité de pas sur une durée de temps, une distance parcourue sur une durée de temps ou une quantité de temps passé par l'utilisateur à effectuer l'exercice ;
la détermination (440), par le dispositif informatique, à un instant actuel et sur la base au moins en partie des informations contextuelles, d'une activité en cours que l'utilisateur est susceptible d'effectuer à l'instant actuel, dans lequel l'activité en cours est une activité sédentaire ou inactive ;
la détermination (450), sur la base de l'activité en cours et de l'exercice recommandé, d'un degré de probabilité que l'utilisateur puisse effectuer simultanément l'exercice recommandé et l'activité en cours ; et
en réponse à la détermination (460) du fait que le degré de probabilité satisfait à un seuil de probabilité, l'émission (470), par le dispositif informatique, d'une notification de l'exercice recommandé.

2. Procédé selon la revendication 1, dans lequel l'obtention de l'indication du mouvement associé au dispositif informatique comprend l'obtention de données de capteur provenant d'un ou plusieurs capteurs (114, 214) du dispositif informatique.

3. Procédé selon la revendication 2, comprenant en outre :
après l'émission de la notification, l'analyse de données de capteur provenant du ou des capteurs et la détermination du fait que les données de capteur correspondent ou non à des schémas de mouvement généralement observés lorsque l'utilisateur effectue l'exercice recommandé ; et
l'ajustement du seuil de probabilité sur la base de la détermination.

4. Procédé selon la revendication 3, dans lequel l'ajustement comprend :
l'abaissement du seuil de probabilité lorsque les données de capteur correspondent à des schémas de mouvement typiquement observés lorsque l'utilisateur effectue l'exercice recommandé ; et
l'augmentation du seuil de probabilité lorsque les données de capteur ne correspondent pas à des schémas de mouvement typiquement observés lorsque l'utilisateur effectue l'exercice recommandé.

5. Procédé selon l'une quelconque des revendications 1 à 4, dans lequel le degré de probabilité est en outre déterminé sur la base d'une ou de plusieurs règles d'exercice stockées.

6. Procédé selon l'une quelconque des revendications 1 à 5, dans lequel l'exercice recommandé est en outre déterminé sur la base au moins en partie d'informations d'activité historiques associées à l'utilisateur et/ou à l'activité en cours.

7. Procédé selon l'une quelconque des revendications 1 à 6, comprenant en outre :
en réponse à la détermination (460) du fait que le degré de probabilité ne satisfait pas au seuil de probabilité, l'abstention de l'émission, par le dispositif informatique, de la notification de l'exercice recommandé ; et
après l'abstention de l'émission de la notification, la réexécution du procédé selon la revendication 1 après une période de temps prédéterminée.

8. Procédé selon l'une quelconque des revendications 1 à 7, dans lequel :
l'activité en cours comprend : conduire ou être passager d'un véhicule en mouvement, être assis ou debout à un bureau, assister à une réunion ou à une conférence, se tenir debout à un arrêt de bus ou à proximité, ou jouer à un jeu électronique sur le dispositif informatique ; et/ou
l'exercice recommandé comprend : se tenir debout, toucher ses orteils, s'étirer, effectuer un nombre suggéré de squats ou marcher.

9. Procédé selon l'une quelconque des revendications 1 à 8, dans lequel les informations contextuelles associées au dispositif informatique comprennent au moins l'un parmi : des données de capteur obtenues à partir d'un ou de plusieurs capteurs (114, 214) du dispositif informatique ; des données d'application obtenues à partir d'une ou de plusieurs applications exécutées sur le dispositif informatique, ou des informations de calendrier associées à l'utilisateur du dispositif informatique (210).

10. Procédé selon l'une quelconque des revendications 1 à 9, dans lequel la détermination de l'activité en cours comprend :
l'envoi, par le dispositif informatique, à un système de suivi d'activité à distance, d'au moins une partie des informations contextuelles associées au dispositif informatique ;
l'interrogation, par le dispositif informatique, du système de suivi d'activité à distance pour obtenir une indication de l'activité en cours ; et
la réception, par le dispositif informatique, de l'indication de l'activité en cours provenant du système de suivi d'activité à distance.

11. Procédé selon l'une quelconque des revendications 1 à 10, dans lequel l'émission de la notification de l'exercice recommandé comprend l'émission, par le dispositif informatique, de la notification pour une émission ultérieure sur un dispositif pouvant être porté,
dans lequel la notification comprend au moins l'une parmi une alerte graphique, une alerte tactile ou une alerte sonore.

12. Dispositif informatique (110, 210) comprenant :
au moins un composant de capteur (114, 214) configuré pour obtenir (400) des informations de capteur indicatives d'un mouvement associé au dispositif informatique ;
au moins un processeur ; et
au moins un module pouvant être utilisé par l'au moins un processeur pour effectuer le procédé selon l'une quelconque des revendications 1 à 11.

13. Système comprenant des moyens pour effectuer le procédé selon l'une quelconque des revendications 1 à 11.

14. Support non transitoire lisible par ordinateur comprenant des instructions qui, lorsqu'elles sont exécutées par un ou plusieurs processeurs matériels, l'amènent ou les amènent à effectuer le procédé selon l'une quelconque des revendications 1 à 11.

15. Programme d'ordinateur comprenant des instructions qui, lorsqu'elles sont exécutées par un ou plusieurs processeurs matériels, amènent le ou les processeurs matériels à effectuer le procédé selon l'une quelconque des revendications 1 à 11.
